# EUROPEAN PATENT SPECIFICATION

(11) **EP 4 516 273 B1**
(45) Date of publication and mention of the grant of the patent: **01.04.2026**
(21) Application number: 23194710.2
(22) Date of filing: 31.08.2023
(51) Int. Cl.: A61F 5/56

(54) **DEVICE FOR TREATING SLEEP APNEA OR OTHER BREATHING DISORDER**
VORRICHTUNG ZUR BEHANDLUNG VON SCHLAFAPNOE ODER ANDEREN ATEMSTÖRUNGEN
DISPOSITIF DE TRAITEMENT DE L'APNÉE DU SOMMEIL OU D'AUTRES TROUBLES RESPIRATOIRES

(43) Date of publication of application: 05.03.2025
(73) Proprietor: Somnicio, Inc., Everett, WA 98208 (US)
(72) Inventor: ABOULHOSN, Walid, 1003 Btekhnay (LB); VALENTINE, Joanna, Mukilteo, WA 98275 (US); ABOULHOSN, Nadia, Everett, WA 98208 (US); YOUMANS, Scott, Bothell, WA 98011 (US); KARAMEH, Fadi, 5447 Ain Zhalta (LB); SALHA, Sary, 5101 Ras el Maten (LB); EL GHOSAYNI, Hadi, 5728 Baakleen (LB)
(74) Representative: Platzöder Patentanwaltsgesellschaft mbH

(56) References cited:
- US-A1- 2023 148 953

## Description

### BACKGROUND OF THE INVENTION

### Technical Field

The present invention generally relates to the field of devices for treating sleep apnea or other breathing disorders. In particular, the present invention can provide a device for treating sleep apnea that is easy to set up and use is compact in size, and may allow for increased comfort for a patient wearing the device while sleeping. Specifically, the present invention provides an oral appliance for treating sleep apnea.

### Background Art

Sleep apnea is a sleep disorder characterized by repeated pauses in breathing during sleep. These pauses can last from a few seconds to a couple of minutes and can occur multiple times throughout the night. Sleep apnea can be of different types. Obstructive Sleep Apnea (OSA) refers to the most common type, caused by a physical blockage or collapse of the airway during sleep, leading to interrupted breathing. The most common symptoms of sleep apnea include loud snoring, abrupt awakenings accompanied by choking or gasping, excessive daytime sleepiness, morning headaches, and difficulty concentrating. This may also cause breathing through the open mouth, which may in turn cause dry mouth and bad breath. Apart from that, open-mouth breathing has an effect on the pH value in the mouth, which may cause damage to the tooth enamel and increase the potential for cavities.

Treatment options for sleep apnea depend on the severity and type of sleep apnea. First of all, a patient may make certain lifestyle modifications that can help alleviate mild cases of sleep apnea. This includes maintaining a healthy weight, regular exercise, avoiding alcohol and sedatives, and sleeping on your side instead of your back. A common treatment for moderate to severe obstructive sleep apnea is Continuous Positive Airway Pressure (CPAP). A CPAP machine delivers a constant stream of air through a mask, which keeps the airway open during sleep. There are also known oral appliances. For instance, certain dental devices can be worn during sleep to reposition the jaw and tongue, helping to keep the airway open. Another treatment is Bi-level Positive Airway Pressure (BiPAP). This machine delivers different pressures for inhalation and exhalation. It can be an option for individuals who have trouble tolerating CPAP. In some cases, surgery may be recommended to remove excess tissue, correct structural abnormalities, or implant devices to help keep the airway open.

CPAP masks are an integral part of the CPAP therapy used to treat obstructive sleep apnea. These masks deliver a continuous flow of pressurized air to keep the airway open during sleep, preventing pauses in breathing. There are several types of CPAP masks available to suit individual preferences and needs. Nasal masks cover the nose and are held in place with straps. They are the most common type of CPAP mask and are suitable for people who breathe through their nose during sleep. Full face masks cover both the nose and mouth. They are suitable for people who breathe through their mouth or have difficulty breathing through their nose.

CPAP masks require a proper fit to maintain an effective seal and prevents air leaks. Most masks have adjustable straps and cushioning for personalized adjustment and comfort. It may take some time to find the right mask and make necessary adjustments to achieve a comfortable fit. Regular cleaning and maintenance of CPAP masks are necessary to ensure hygiene and prolong their lifespan. Overall, CPAP masks tend to be bulky, may be inconvenient in use and may affect the patient's sleep, e.g., because a tube connection to the CPAP machine may restrict movements of the patient.

Devices that secure the lips or jaws of a patient to prevent pauses in breathing during sleep are typically referred to as oral appliances. These devices are used as an alternative treatment option for certain individuals with obstructive sleep apnea (OSA) who are unable to tolerate or prefer not to use a CPAP machine. Oral appliances work by repositioning the jaw, tongue, and other oral structures to help maintain an open airway during sleep. By advancing the lower jaw (mandible) slightly forward, the oral appliance helps to prevent the collapse or obstruction of the airway that leads to breathing pauses. Such mandibular advancement devices (MADs) resemble mouthguards or dental retainers. They consist of separate upper and lower splints that fit over the teeth and are connected by hinges. The lower splint is adjusted to hold the jaw in a forward position, thus opening the airway. Typically, a dentist will assess the patient's dental and oral health, take impressions or digital scans of the teeth, and design a custom-fitted oral appliance, which may be a hurdle for the patient and may lead to high costs for the device.

There are also devices available that hold the lips of a patient closed together. Such devices resemble an adhesive tape that is placed over the mouth. While a little hole may allow breathing through the mouth, such devices may cause discomfort. Apart from that, they are less effective for preventing collapse or obstruction of the airway because keeping the lips closed generally has hardly any effect on the actual position of the jaws, particularly with regards to mandibular advancement. US 2023/148953 A1 describes a device for treating sleep apnea according to the preamble of claim 1.

### SUMMARY OF THE INVENTION

It is an object of the present invention to provide an improved device for treating sleep apnea. Specifically, an improved sleep apnea device shall be provided that effectively can prevent collapse or obstruction of a patient's airway during sleep. The device shall be designed to be easily set up and used by the patient and to be compact in size to reduce discomfort for the patient during sleep when wearing the device.

A solution to this problem is provided by the teaching of independent claims. Various preferred embodiments of the present invention are provided by the teachings of the dependent claims.

According to one aspect, a device for treating sleep apnea is provided. The device comprises an upper portion, a lower portion, and an intermediate portion. The upper portion is configured to be placed on at least one tooth of the upper jaw of a patient, and the lower portion is configured to be placed on at least one tooth of the lower jaw of the patient. The intermediate portion is configured to couple the upper and lower portions together to maintain a fixed position of the upper and lower jaws when placed on the teeth of the upper and lower jaws of the patient. The device further comprises at least one fastening member, the fastening member being configured to engage at least one interdental space of each the upper and lower jaws to fasten the upper and lower portions to the teeth of the upper and lower jaws, respectively.

The device of the present invention, thus, provides an improved oral appliance for treating sleep apnea, specifically obstructive sleep apnea (OSA), that is able to maintain the upper and lower jaws of the patient in a fixed position to keep the airway open. It will be appreciated that the "fixed position" is not necessarily a totally fixed position but may allow movement to a certain extent within certain limits which does not affect effectiveness of the sleep apnea treatment, i.e., as long as it is ensured that the jaws are maintained in such a position that prevents obstruction of the airway. Thus, the "fixed position" may also be referred to as "relative position". Specifically, in some cases, the device may be able to maintain a fixed position of the jaws where the lower jaw is prevented from falling in a backward position (relative to a natural position). The device may generally also be referred to as "oral device" or "intra-oral device" and provides a treatment device for open airway therapy (OAT). Keeping the upper and lower jaws in a fixed position and, thus, substantially closed together, also can prevent the tongue from falling backwards (which would cause obstruction of the airway).

The device of the present invention also provides an improved device for treating mouth breathing, snoring, or other breathing disorder for the same reasons. Thus, for the sake of simplicity all are referred to as "sleep apnea" throughout this document. This means that the term "sleep apnea" shall refer to "sleep apnea, mouth breathing, snoring, or other breathing disorder".

Specifically, in some cases, the device may be able to maintain a fixed position of the jaws where the lower jaw is in a slightly forward position (relative to a natural position). Thereby, the device may also be referred to as a mandibular advancement device (MAD). Such position of the jaws specifically may tighten and thereby prevent over-relaxation of the soft tissue and muscles of the upper airway (including the so-called soft palate) which may lead to obstruction of the airway thereby causing pauses in breathing or shallow breaths during sleep. The device may also be suitable to treat snoring because the tightening created by the device also prevents the tissues of the upper airway from vibrating as air passes over them, which is the most common cause of snoring.

The device can be easily set up and secured in the patient's mouth (more specifically to the teeth) by inserting the fastening member into at least one interdental space. Thus, the device can fit various sets of teeth without the need of custom fitting it for a specific patient. Finally, this provides a device at relatively low costs. The device is convenient to wear because the provision of the fastening member may allow a design without clamping the teeth too tightly, which might cause discomfort. Furthermore, the lips of the patient are free such that breathing through the mouth is still possible.

The terms "upper", "lower" and "intermediate" are used for distinguishing the portions of the device, in particular relative to a patient's mouth when worn. Thus, it will be appreciated that the position and orientation may vary when the device is not worn by a patient or when the patient moves. The upper jaw is also referred to as maxilla, while the lower jaw is also referred to as mandible.

The terms "first", "second", "third" and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Where the term "comprising" or "including" is used in the present description and claims, it does not exclude other elements or steps. Where an indefinite or definite article is used when referring to a singular noun e.g., "a" or "an", "the", this includes a plural of that noun unless something else is specifically stated.

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In the following, preferred embodiments of the device are described, which can be arbitrarily combined with each other or with other aspects of the present invention, unless such combination is explicitly excluded or technically impossible.

In some embodiments, the upper and lower portions each have at least one securing means for securing the fastening member to the upper and lower portions, respectively. The securing means may comprise at least one of an opening, an eye, a pin, and a hook for engagement with the fastening member. There may be provided holes through which the fastening member can be threaded (or is threaded). One or more of the holes may be elongate (in a lateral direction) to allow reception of the fastening member in interdental spaces for different sizes of teeth. A ratchet mechanism or the like may be provided in the holes to allow securing and also tightening the fastening member.

In some embodiments, the fastening member comprises at least one piece of floss. The floss may be preloaded, particularly secured in or by the aforementioned securing means. This facilitates use and application of the device for the patient as there is no need to manually thread (or otherwise secure) the floss in the portions of the device. Rather than floss, the fastening member may be of a different elongate type, such as a wire, band, cable, or the like. For instance, the fastening member may comprise at least one nitinol wire or elastic band. In particular, the fastening member(s) may be made of any suitable material, including plastics, rubber, or metal, i.e., not necessarily materials typically used for (dental) floss (such as Nylon, Polyethylene or PTFE). The fastening member may have any cross-sectional shape that is suitable to fit into an interdental space, such as round or rectangular. Alternatively (or in addition) the fastening member may comprise at least one staple or at least one pin, which may penetrate at least one interdental space, just like, e.g., floss. Fastening could further alternatively (or in addition) be done using an adhesive material such as dental adhesives.

In some embodiments, the fastening member comprises one or more strands of floss, one or more loops of floss, or a combination thereof. Furthermore, the fastening member may comprise at least one piece of floss separately for each of the upper and lower portions, at least one piece of floss together for both of the upper and lower portions, or a combination thereof. In particular, strands or loops of floss may be provided to extend only through one interdental space or through more than one. There may be one strand or loop of floss that extends through interdental spaces in the upper and lower jaws such that by tightening the dental floss additional fixation of the upper and lower jaws may be achieved.

In some embodiments, the upper and lower portions are formed as dental splints that are each configured to receive teeth of the upper jaw and lower jaw, respectively. Each splint may extend along a section of the upper and lower jaws, i.e., it may receive more than one tooth. Such a section may include one or more anterior teeth (front teeth) one or more molar teeth (back teeth) or both. The term "sleep apnea mouthguard" may be used for the device. The splints may have a substantially V or U-shaped cross-sectional profile that is suitable for receiving the teeth or within one or more interdental spaces. The V or U shaped geometry may also be formed to provide mild compressive force due to the material of construction onto teeth providing retention for retention of the splints. A snapping or mechanical mechanism may be provided, which allows the splints to snap onto the teeth. For instance, the "legs" of a V or U-shaped profile may be in a wide configuration that may close ("snap") towards the surfaces of the teeth (e.g., using a snap mechanism known from so-called "snap wraps"). Thereby, a clamping force may be created that may support the hold of the splints on the teeth.

In some embodiments a moderate release or moisture activated biocompatible adhesive may be used that retains or provides retention of the splint to the teeth while enabling user easy removal upon awaking.

In some embodiments, the splints each have a retaining structure on an inner side thereof, the inner side facing at least one tooth when placed on the tooth with the retaining structure engaging a surface of at least one tooth or at least one interdental space. The retaining structure may have at least one of one or more ridges, barbs, knobs, pins, or the like in a horizontal or vertical arrangement. In this way, the risk of the splints slipping off the teeth can be reduced or, in other words, the grip and hold of the splints on the teeth can be improved. The splints do not have to be custom fitted as the retention structure may be resilient and adapt to the teeth of different patients.

Rather than comprising splints, the upper portion, the lower portion, and the intermediate portion together form a plate-like or pad-like element in alternative embodiments. Speaking in more general terms, while splints are typically configured to extend around the teeth on both opposite sides (i.e., vestibular (labial / buccal) and oral (palatal / lingual)), devices of such alternative embodiments may engage only one of the sides of the teeth. For instance, a plate or pad-like element in the region of the upper and lower anterior teeth may be provided which is secured to the teeth by means of floss to maintain the fixed position of the upper and lower jaws. It will be appreciated that the term "plate" or "pad" does not necessarily mean to have a totally flat shape but may comprise a curvature suitable to adapt one or more teeth. In particular, the plate or pad-like element may be flexible such it adapts to the dental arches of the upper and lower jaws. While floss may be sufficient to hold the device in place, the upper and lower portions may have engagement means configured for engaging the surface of at least one tooth, e.g., suction cups or the like.

In some embodiments, the upper portion, the lower portion, and the intermediate portion are formed as an integral piece. This may facilitate use of the device because no assembly in the mouth is necessary. An integral design also prevents individual parts from being lost.

In some embodiments the intermediate portion comprises at least one elongate connection member connecting the upper and lower portions. For instance, the upper and lower portions may be connected to each other by a cable or band-like element. The elongate connection member may be flexible or semi-rigid to allow movements of the upper and lower jaws to a certain extent as long as the function as described above for treating sleep apnea is not affected negatively.

In some alternative embodiments, the upper and lower portions are formed as separate pieces, wherein the intermediate portion comprises a first portion attached to the upper portion and a second portion attached to the lower portion, the first and second portions being separate from each other and each accommodating a magnet such that the first and second portions are attachable to each other by magnetic force. In this way a two-piece design of the device can be provided, wherein securing the fixed position of the upper and lower portions and thereby the upper and lower jaws is achieved by the magnets.

In some embodiments, the device further comprises a locking member configured to engage the intermediate portion or the fastening member to secure the fixed position of the upper and lower jaws. For instance, the locking member may be a plate-like element. Such plate-like element may be provided with an opening or other receiving structure to engage the intermediate portion to tighten the device. For instance, the intermediate portion may comprise a cable (see above) threaded through the locking member, which can be pulled to tighten.

In some embodiments, the fastening member (e.g., floss) has an anchoring mechanism for anchoring in an interdental space. The anchoring mechanism may comprise one or more anchoring members arranged along a length of the fastening member or at an end thereof and may comprise a ball or knot (or other kind of indentation) or a barb or hook. The anchoring mechanism improves the hold of the fastening member in an interdental space by preventing slipping out of the interdental space. A fastening member with an anchoring mechanism may also be used to tighten the device to the teeth. The anchoring mechanism may also be configured to swell when it comes in contact with saliva. For instance, swelling floss may be used, which may improve its hold in the interdental spaces.

In some embodiments, the device further comprises a tongue holding portion that is attached or attachable to at least one of the upper and lower portions, the tongue holding portion being configured to be placed on at least a portion of the patient's tongue to hold the tongue and prevent the tongue from moving towards the patient's throat. This may further improve treatment of sleep apnea. For instance, the tongue holding portion may be attached to the tongue by a suction force, where the tongue holding portion may be provided in the form of a suction cup that can be placed over the tip of the tongue. In order to hold the tongue in a forward position, the tongue holding portion can attach to the upper and/or lower portions by means of magnets. Thus, the tongue is held in placed but can be also easily released if desired.

In some embodiments, the upper portion and the lower portion are formed from a flexible material, such as rubber, silicone, or the like. It will be appreciated that the material is preferably a medical grade suitable material for use in the mouth. Any of the here described embodiments may be at least partially, or even completely disposable. This means that the device may be configured for single use, i.e., for one night.

In some embodiments, the device further comprises at least one sensor for measuring at least one parameter in the patient's mouth. In this way, the device is not only suitable for open airway therapy but also for monitoring (body) parameters that may occur in the context of sleep apnea. The parameters may comprise at least one of temperature, oxygen level, pH-value, breathing pattern, and snoring. The pH-value of the saliva may be an indicator for mouth breathing as the pH-value will slightly increase (typically from slightly acidic to slightly basic) when the patient breathes through the mouth. Measurement data can be stored and read out by another device, such as a mobile device, e.g., smartphone, a distinct reading device, or other user device, or can be directly sent to another device. Reading or sending may use a wired or wireless connection, such as Bluetooth or NFC. The sensor may be arranged in the intermediate portion (or locking portion, if applicable). Thereby, the sensor can be positioned between the upper and lower jaws, e.g., in front of the front teeth in direct contact with the cheeks, the gum, or the tongue which promotes measurement of several biometric signals. This may improve the measurement results, particularly with regards to the oxygen, temperature, PH, breathing frequency, and other biometrics.

According to another aspect, a set may be provided, comprising a plurality of devices for treating sleep apnea as described above, wherein at least one of the devices may have a sensor as explained above. This may be particularly advantageous if the devices are configured as single-use (disposable) devices. For instance, a set may comprise devices for several days or weeks, e.g., one week or one month, wherein only some of the devices may be provided with a sensor, e.g., one per week, five per month or the like. On the one hand, this may save costs. On the other hand, it may be sufficient to measure the aforementioned body parameters only once a week (i.e., only one night per week). It may also be envisioned to provide the sensor separately, and to attach it to a device, such that measurements can be made as often as needed.

It will be appreciated that explanations, embodiments, and advantages described above in connection with one aspect may similarly apply to any other aspect of the invention.

### BRIEF DESCRIPTION OF THE DRAWINGS

Illustrative embodiments are described below with reference to the drawings. In the interest of clarity, not all features of an actual implementation may be described in this specification. It will of course be appreciated that in the development of any such actual embodiment, numerous implementation-specific decisions might be made to achieve the developers' specific goals, such as compliance with system-related and business-related constraints, which will vary from one implementation to another. In the drawings:
Figs. 1a - 1d show different views of an embodiment of a device for treating sleep apnea comprising two splints coupled by flexible cables;
Figs. 2a - 2d show different views of an embodiment of a device for treating sleep apnea comprising two splints coupled by a flexible cable with a locking member;
Fig. 3a shows an embodiment of a device for treating sleep apnea comprising two splints coupled by magnets;
Fig. 3b shows an embodiment of a device for treating sleep apnea comprising two splints coupled by magnets similar to that of Fig. a but with magnets in different positions;
Figs. 4a and 4b show different views of another embodiment of a device for treating sleep apnea comprising two splints coupled by magnets;
Figs. 5a and 5b show two configurations of an embodiment of a device for treating sleep apnea comprising two snapping splints coupled by a rubber band;
Fig. 6 shows an embodiment of a device for treating sleep apnea comprising a flexible pad;
Fig. 7 shows different views (cross-section and side view) of a securing means for securing the fastening member;
Fig. 8a shows an embodiment of a device for treating sleep apnea comprising a locking plate;
Figs. 8b and 8c show details for the embodiment of Fig. 8a;
Figs. 9a and 9b show different embodiments of a device for treating sleep apnea comprising a fastening member with anchoring means in different configurations;
Fig. 10 shows different configurations of another embodiment of a device for treating sleep apnea comprising a fastening member with anchoring means;
Fig. 11 shows a fastening member in the form of a nitinol wire in different configurations;
Fig. 12 shows a fastening member in the form of a swelling floss in different configurations;
Fig. 13 shows steps of installing a fastening member having a knot mechanism;
Fig. 14 shows a tool for installing a device for treating sleep apnea; and
Figs. 15a and 15b show an embodiment of a device for treating sleep apnea comprising an elastic band secured to the teeth in different configurations;
Fig. 16 shows an embodiment of a device for treating sleep apnea comprising a tongue holding portion.

### DESCRIPTION OF EMBODIMENTS

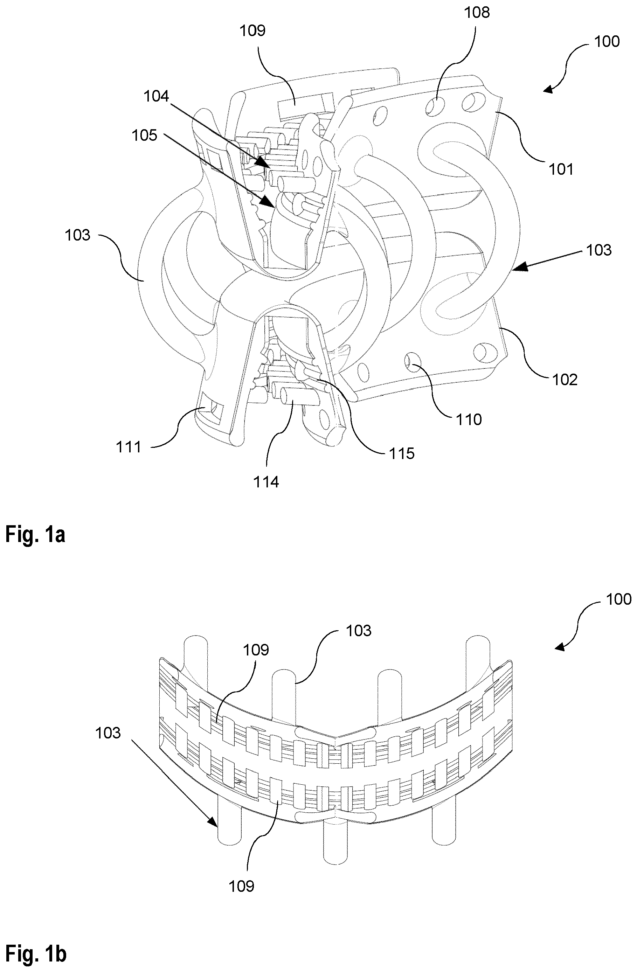

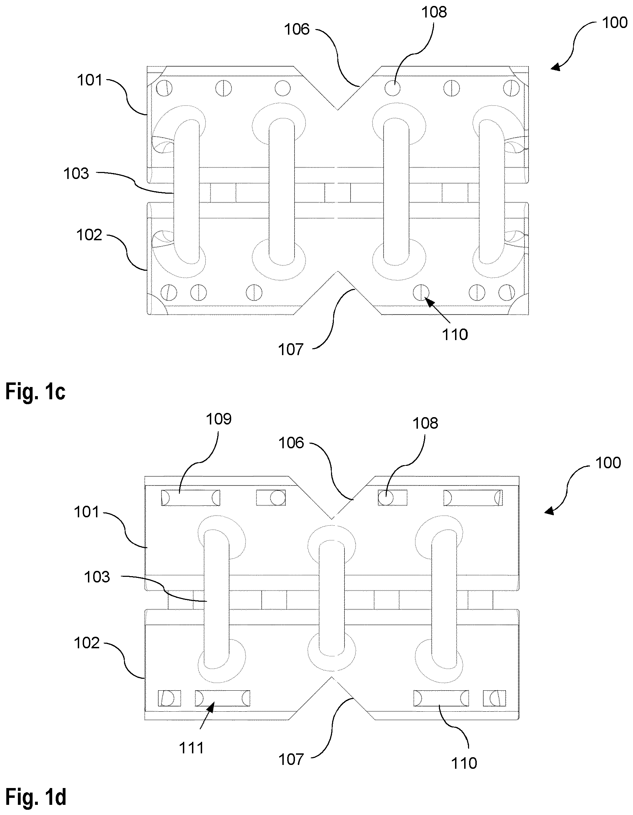

The drawings show various embodiments of a device for treating sleep apnea. More specifically, the device is configured for treating obstructive sleep apnea (OSA), which is caused by obstruction of the airway during sleep. The device of the present invention aims at maintaining a fixed position (or "relative position") of the upper and lower jaws of patient. That means, by fixing the jaws together, the device may thereby help to prevent the collapse or obstruction of the airway that leads to breathing pauses. Such mandibular advancement devices (MADs), particularly those shown in Figs. 1 to 3, resemble mouthguards or dental retainers and may also serve as bite splints as an additional function.

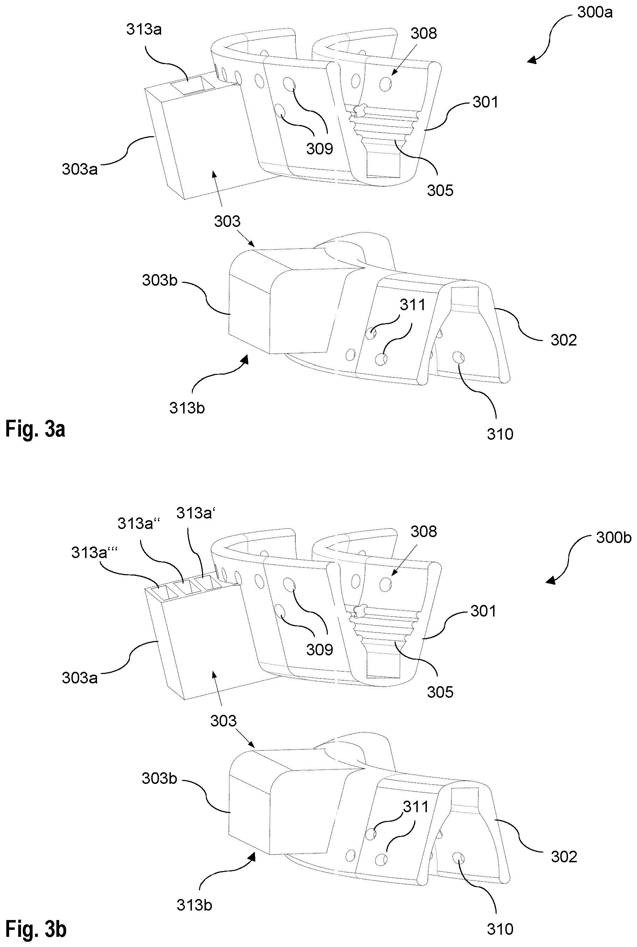

As will be described in further detail below, with the device of Fig. 3b a fixed position of the jaws could be maintained in which the lower jaw (mandible) is held in a (slightly) forward position. This may enhance the effectiveness of the device. However, it will be appreciated that it is not necessary to advance the lower jaw to achieve the advantages of the present invention, but it is sufficient to maintain a fixed position of the upper and lower jaws (with the device of Figs. 1a-d, 2a-d and 3a) to prevent the lower jaw from falling backwards (while possibly opening the mouth).

An embodiment of the device 100 is shown in a perspective view in Fig. 1a, a view from above (or below, which is basically the same view) in Fig. 1b, a view from the back in Fig. 1c and a view from the front in Fig. 1d. The device 100 has an upper portion 101 and a lower portion 102, both in the form of splints that are configured to fit on teeth of the upper and lower jaws, respectively. The upper and lower portions 101, 102 are coupled to each other by means of an intermediate portion 103, which is here in the form of flexible or semi-rigid cables, three in the front and four in the back (the number, position, diameter and arrangement of the flexible cables may vary). The upper portion 101, lower portion 102, and intermediate portion 103 are integrally formed, i.e., made of a single piece of material, such as an elastic plastics material. However, it may also be possible that the upper and lower portions 101, 102 are made from a different (e.g., harder, softer, more rigid) material than the intermediate portion 103. The intermediate portion 103 (flexible cables) is flexible but sufficiently rigid to maintain a fixed position of the upper and lower jaws, while allowing movement to a certain extent. This allows adaptation to different jaw positions ("occlusions") of different patients. The rigidity of the cables 103 may also provide an element of formability, which could be formed by the user using a provided tool to determine the relative position of the upper and lower jaw. Splints 101 and 102 may have a predetermined or adjustable fore/aft offset to promote mandibular advancement further reducing sleep apnea or snoring by varying the construction of intermediate portion 103.

The splints forming the upper and lower portions 101, 102 have a substantially V-shaped cross-section that is sized and shaped to receive teeth of the upper and lower jaws. As particularly shown in Fig. 1b, the upper and lower portions 101, 102 have a curved shaped to receive tooth rows of the upper and lower jaws, respectively. A retaining structure having barbs 104, 114 and ridges 105, 115 is provided to engage the either the surfaces of the teeth or the interdental spaces to thereby support holding the device 100 in place. In this embodiment there is a single row of barbs 104, 114 one from the front, one from the back, for each of the upper and lower portions 101, 102 (i.e., in total four rows). The ridges 105, 115 extend in a lateral direction in parallel to the rows of barbs 104, 114, however they could also be positioned vertically to aid with mechanical retention. Apart from that, recesses 106, 107 are provided to avoid interference with the frenula of the lips. Splints 101 and 102 could also be coated with a moderate release biocompatible adhesive that provides adequate adhesion to retain to teeth yet promote user removal upon awaking.

Intermediate portion 103 may have flexible/semi-rigid cable assembly design and geometry to support tongue depression or restraint during use.

The upper and lower portions 101, 102 are provided with holes 108, 109, 110, 111 for receiving a fastening member (not shown here, but see description below), in particular floss. The fastening member helps to secure the device 100 to the teeth by passing through interdental spaces. While the holes 108, 110 in the back (the posterior side) are round, the holes 109, 111 in the front (the anterior side) are elongated in a lateral direction. In this way, the device 100 is suitable for various patients without the need for customized holes. The floss also prevents or at least reduces lateral movement of the jaws.

Splints 101 and 102 are shown separated with intermediate section 103 in Figure 1a, but they could also be constructed as a single unit piece with predefined fore-aft construction to provide mandibular displacement with one or more of the above-outlined features included.

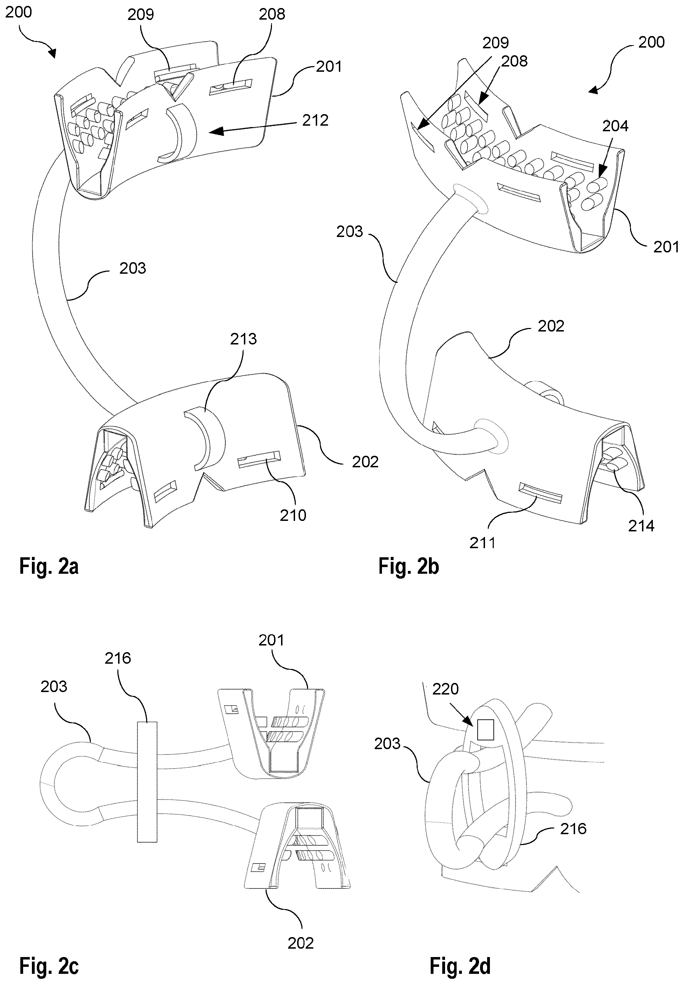

Another embodiment of the device 200 for treating sleep apnea is shown in a perspective view in Figs. 2a and 2b, while Figs. 2c and 2d show detailed views of the device 200. The device 200 is similar to the device 100 described above. Thus, the respective description of Figs. 1a to 1d applies where applicable. The major difference is the design of the intermediate portion 203. Rather than having a plurality of flexible cables, a single and longer flexible cable is provided that couples the upper and lower portions 201, 202 together. In order to maintain a relative position of the jaws, a locking member 216 can be pushed onto the flexible cable when bent as shown in Figs. 2c and 2d. The locking member 216 can be advanced towards the teeth to tighten the device 200, i.e., to hold the upper and lower portions 201, 202 together. The patient can thereby control how tight the device 200 is secured. Further securing can be achieved by threading floss (or other flexible and elongated element) through hooks 212, 213 on the back side of the upper and lower portions 201, 202. In order to support hold on the teeth barbs 204, 214 are provided on the inner side of the upper and lower portions 201, 202. Here, two rows of barbs 204, 214 are each provided (in total eight) but no ridges. Floss can be threaded through holes 208, 209, 210, 211, all of which have an elongated shape in this embodiment.

The device 200 may comprise sensor 220 for measuring parameters in the patient's mouth. Thus, the device 200 may also function as a device for monitoring the sleep of the patient by measuring parameters in the mouth. For instance, the pH-value of the saliva may be an indicator for mouth breathing as the pH-value will slightly decrease (typically from slightly basic to slightly acidic) when the patient breathes through the mouth. A rise of the pH-value may cause damage to the tooth enamel increasing the risk of cavities. The parameters may comprise a temperature, an oxygen level, heart rate, or a pH-value (or various combinations thereof, including further parameters that may be measured in the mouth). It may also be envisioned to detect a breathing pattern and/or snoring, which may be an indicator for the severity of the sleep apnea. It will be appreciated that the obtained measurement data can be stored, read out, sent, and further processed, and analyzed as required. Reading or sending may use a wired or wireless connection, such as Bluetooth or NFC.

With the sensor 220 being arranged in the locking member 216 it can be positioned between the upper and lower jaws, e.g., in front of the front teeth, which puts the sensor 220 in the flow of the breathing air. This may improve the measurement results, particularly with regard to the oxygen content in the breathing air. While in this embodiment the sensor 220 may be preferably arranged in the locking member 216, it will be appreciated that a sensor 220 may be provided in any of the embodiments, particularly in an intermediate portion of the respective device. More generally in any portion of the device that is suitable to put the sensor in the breathing air and in direct contact with the cheek, tongue, or gum, particularly if the sensor shall measure an oxygen level in the breathing air and other biometric data that may be of value.

Furthermore, for any embodiment described herein, a set may be provided, comprising a plurality of devices for treating sleep apnea, wherein at least one of the devices may have a sensor as explained above. This may be particularly advantageous if the devices are configured as single use (disposable) devices. For instance, a set may comprise devices for several days or weeks, e.g., one week or one month, wherein only some of the devices may be provided with a sensor, e.g., one per week, five per month or the like. On the one hand, this may save costs. On the other hand, it may be sufficient to measure the aforementioned body parameters only once a week (i.e., only one night per week). It may also be envisioned to provide the sensor separately, and to attach it to a device, such that measurements can be made as often as needed.

Fig. 3 shows a perspective view of still another embodiment of a device 300 for treating sleep apnea. It is similar to those described above with reference to Figs. 1 and 2. Thus, the above description applies where applicable. However, the intermediate portion 303 is different. Rather than directly connecting the upper and lower portions 301, 302, the intermediate portion 303 in the embodiment of Fig. 3 has two separate parts 303a, 303b. Each part 303a, 303b forms a waterproof compartment accommodating a magnet, such that the upper and lower portions 301, 302 can be held together by magnetic force to achieve maintaining a relative position of the upper and lower jaws. Structures 303a and 303b could be designed to provide for adjustment of the relative magnet locations to provide varying degrees of mandibular advancement based on the patients' need and experience. Further, structures 303a and 303b could be placed on the interior (inner curvature) of splints 301 and 302 to provide the same magnetic retention and adjustable mandibular displacement, but could also provide tongue retention and house sensors for biometric measurement from the tongue. The retaining structure on the inner surface of the upper portion 301 only include ridges 305 running in a lateral direction. Holes 308, 309, 310, 311 for receiving floss are provided. However, rather than providing elongated holes, only round holes are provided. The greater number of holes 308, 309, 310, 311 (compared to the embodiments described above) and offset arrangement allows using the device 300 for various patients.

It is noted that the structures of the splints described with reference to Figs. 1 to 3, in particular the configurations and types of holes for receiving floss and the configurations of the retaining structure including barbs and ridges may be variable and interchangeable and may also be combined throughout the embodiments. Additional hold may be achieved by adhesive provided in the splints that may or may not be activated by saliva.

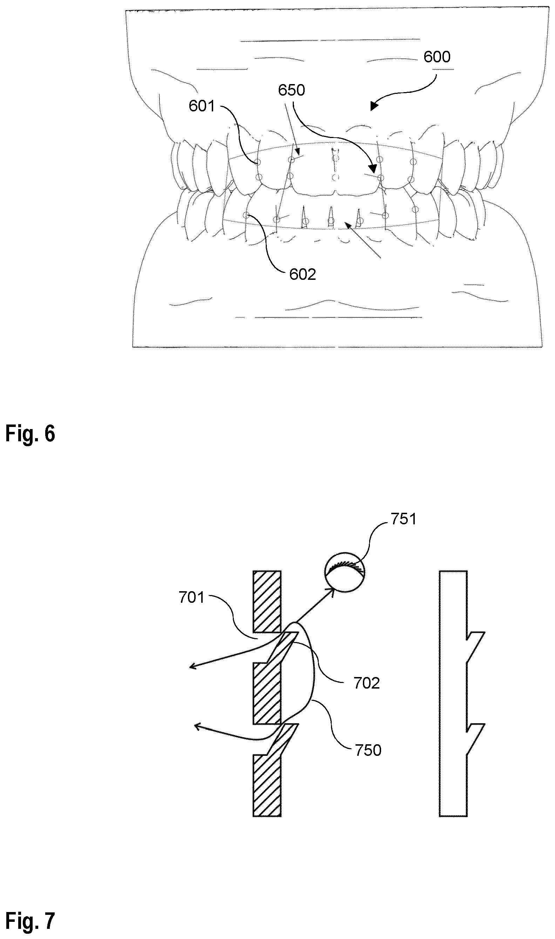

It is further noted that the fastening member, such as floss, is not shown in Fig. 1 to 3 for clarity purposes. Examples for fastening members will be explained below particularly with respect to Figs. 6 to 15. Thus, features described in connection with Figs. 6 to 15, particularly as far as they concern the fastening member, may apply to the embodiments of Fig. 1 to 3. More specifically, the devices of Figs. 1 to 3 may be used with any of the examples of fastening members described below.

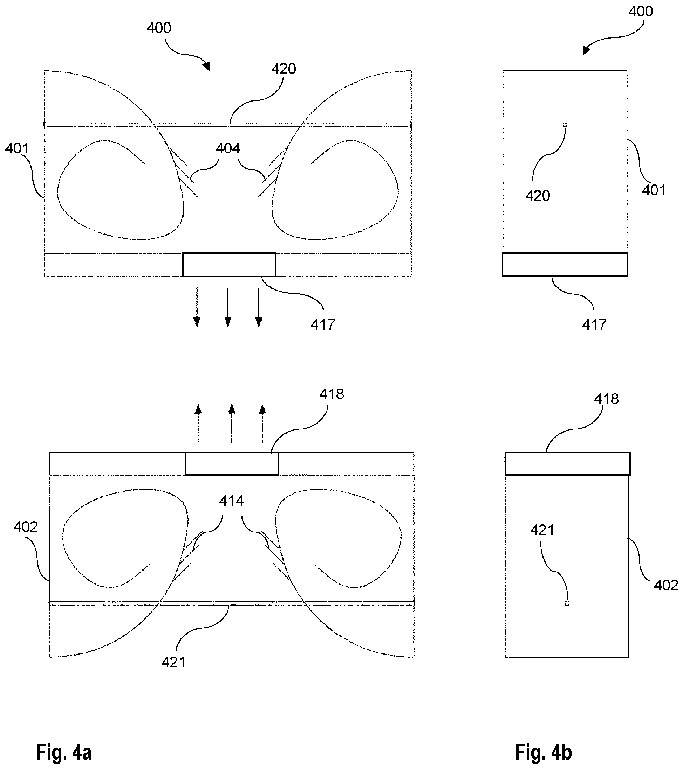

Another embodiment of a device 400 for treating sleep apnea is shown in Fig. 4a (cross-sectional view) and 4b (front view). Similar to the splints as described above, the device of Figs. 4a and 4b is configured to receive teeth of the upper and lower jaws. However, as can be seen from Fig. 4b, the device 400 is smaller and may extend, e.g., only over the front teeth (incisors). A single strand of floss 420, 421 is provided in each of the upper and lower portions 401, 402 to extend through one interdental space in each of the upper and lower jaws. Nevertheless, the device 400 may also be wider to span more teeth. Accordingly, more than one floss line may be provided. Further support is provided by a biased configuration of the upper and lower portions 401, 402 that allows to apply a clamping force to the teeth. Barbs 404, 414 are provided for further support, which may have a rough surface and/or may be sticky to attach to the surface of the teeth. Magnets 417, 418 are provided to hold the upper and lower portions 401, 402 together. Here the magnets 417, 418 are accommodated in ends of the upper and lower portions 401, 402 facing each other when the device 400 is worn and attract each other to secure a relative position of the upper and lower jaws. The magnets 417, 418 may be movable to ensure that they meet optimally or to provide mandibular advancement.

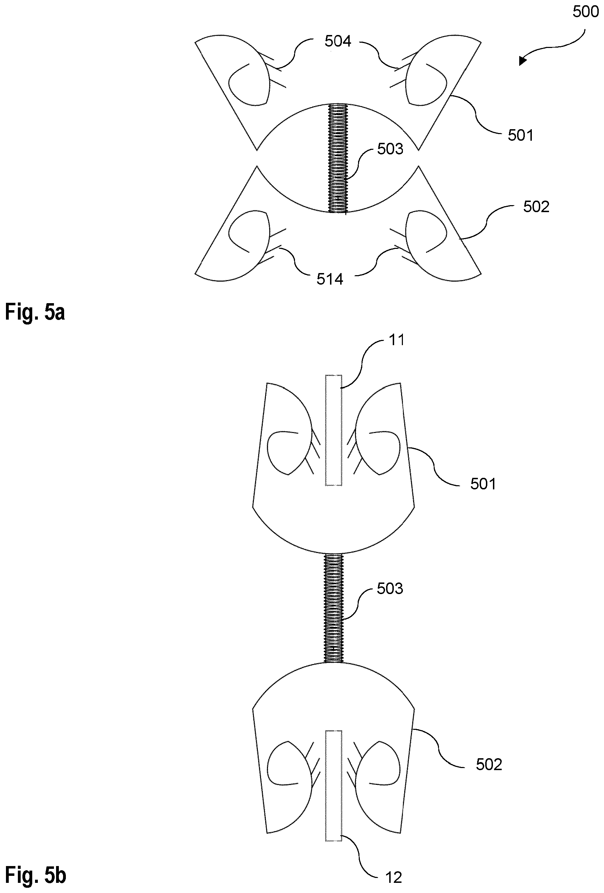

Another embodiment of a device 500 for treating sleep apnea is shown in Figs. 5a and 5b. The device 500 is configured to snap onto the teeth 11, 12. Fig. 5a shows an open configured, in which the device 500 can be placed on the teeth 11, 12. When the patient bites the device 500, the teeth 11, 12 push into the upper and lower portions 501, 502, respectively, the upper and lower portions 501, 502 snap into a closed configuration shown in Fig. 5b to clamp the teeth 11, 12. The biased shaped and barbs 504, 514 similar to the device 400 of Figs. 4a and 4b further supports hold on the teeth 11, 12, and the device 500 can have a similar (narrow) lateral dimension. The intermediate portion 503 is here in the form of a rubber band. Just like in the above-described embodiments, the rubber band helps to maintain a relative position of the upper and lower jaws when the device 500 is worn, while allowing the patient to move the jaws to a certain extent as explained above.

While in the embodiments described above, the upper and lower portions of the device enclose the teeth on the front side and back side, in the embodiments described below, the device is arranged only on one side of the teeth, preferably the front side. In particular, in most of the following embodiments a plate-like or pad-like device is provided, which is adapted to engage to the teeth of the upper jaw and the teeth of the lower jaws (thereby forming an "upper portion" and a "lower portion", which are integrally connected by an "intermediate portion", i.e., the middle portion of the plate-like or pad-like device).

In the embodiment of Fig. 6 the device 600 comprises a flexible pad, with holes 601, 602 in the upper and lower portion for receiving fastening members 650. In the upper portion, two holes 601 are provided for each interdental space, such that the device is adapted for various patients to maintain a relative position of the upper and lower jaws during sleep. In this embodiment, the fastening members 650 are formed as staples, which are substantially rigid. However, floss may also be used.

Fig. 7 shows a possible hole configuration that may help securing floss 750, which may apply for any embodiment using floss as the fastening member(s). A bar or bridge 702 protrudes into the holes 701 such that only a small opening remains that may clamp the floss 750. For further hold, the floss 750 may be provided with barbs 751. In this way, the floss 750 can be prevented from slipping through the holes 701 backward after tightening.

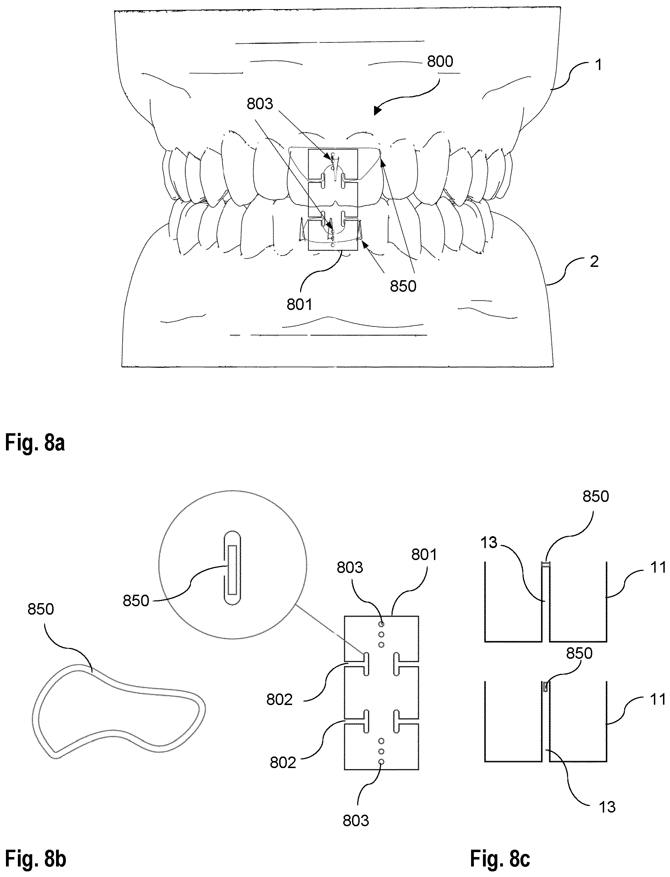

Fig. 8a show another embodiment of a device 800 for treating sleep apnea, while in Fig. 8b and 8c details of the device 800 are shown. The device 800 comprises a plate-like member 801 to maintain a relative position of the upper and lower jaws. The fastening members 850 are formed by elastic rings (e.g., rubber band), which are to be inserted into two interdental spaces and guided around the respective teeth 10. Thus, the plate-like member 801 has slotted openings 802 such that the elastic rings 850 can be inserted. The elastic rings 850 have a rectangular cross-section such that in combination with the T-shaped slotted openings 802 the elastic rings 850 are securely held in the plate-like member 801. In order to tighten the elastic rings 850, hooks 803 are provided on the front side of the plate-like member 801. Here, three hooks 803 are provided in an upper portion and a lower portion of the plate-like member 801 to provide three levels of tension for holding the upper and lower jaws 1, 2 together, which also allows the device 800 to be used for various patients without needing to customize it. The elastic rings 850 can be turned by 90 degrees in an interdental space 13 to lock. While in this embodiment the plate-like member 801 has a width that extends only over two front teeth 10, it may be wider for distributing the force on the teeth more evenly. It will be appreciated that the plate-like member 801 may also be placed on other teeth. This may also apply to other embodiments disclosed herein having such or similar plate-like member.

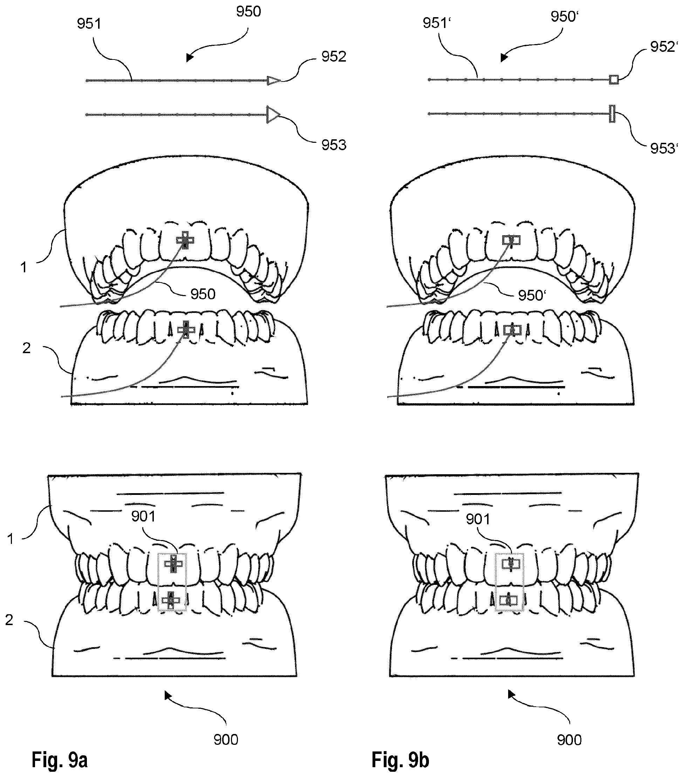

Figs. 9a and 9b show embodiments of a device 900 for treating sleep apnea, in which the fastening members 950 (floss or other elongate fastening member) have anchoring members 951, 952, 953. In the example of Fig. 9a, the floss 950 has arrowhead-like tips 952, 953. The arrowhead-like tip 952 at a "leading end" has a smaller diameter, i.e., this end can be pushed through an interdental space (and hole of a plate-like member 901), while the arrowhead-like tip 953 at a "rear end" has a larger diameter that acts a stopper at the plate-like member 901 when pulled therethrough. Furthermore, ball-shaped anchoring members 951 (are other suitable indentation) are provided along the along such that the fastening member 950 can be tightened.

A similar embodiment is shown in Fig. 9b. The only difference is the shape of the end members 952', 953' of the fastening member 950' (likewise having indentation 951' along its length). Rather than having a pointed shape like the arrowhead-like tips 952, 953 of the embodiment of Fig. 9a, "blunt" ends are provided, such as block-like or plate-like end members 952', 953'. It will be appreciated that any combination of the end members 952, 953, 952', 953' of Figs. 9a and 9b (or other shapes, such as ball-shaped members or the like) may be used (provided that one is small enough for insertion and the other one is large enough to act as a stopper).

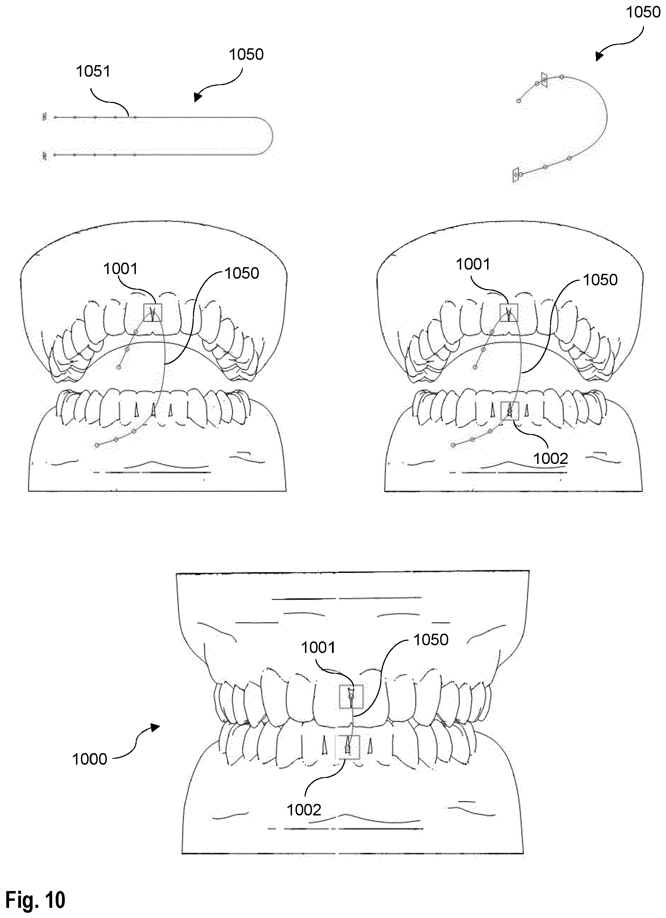

Another embodiment of a device for treating sleep apnea is shown in Fig. 10. Rather than the integrally formed plate-like member as shown in Fig. 9, two separate plate-like members 1001, 1002 are provided. Similar to the embodiment of Fig. 9, the floss 1050 has indentations 1051 along its length. Fig. 10 illustrates the steps of inserting the plates 1001, 1002 and floss 1050. In the bottom illustration of Fig. 10 the final phase is shown, i.e., when the floss 1050 has been tightened, such that the upper and lower jaws 1, 2 are in the relative position that is intended for treating sleep apnea as described herein.

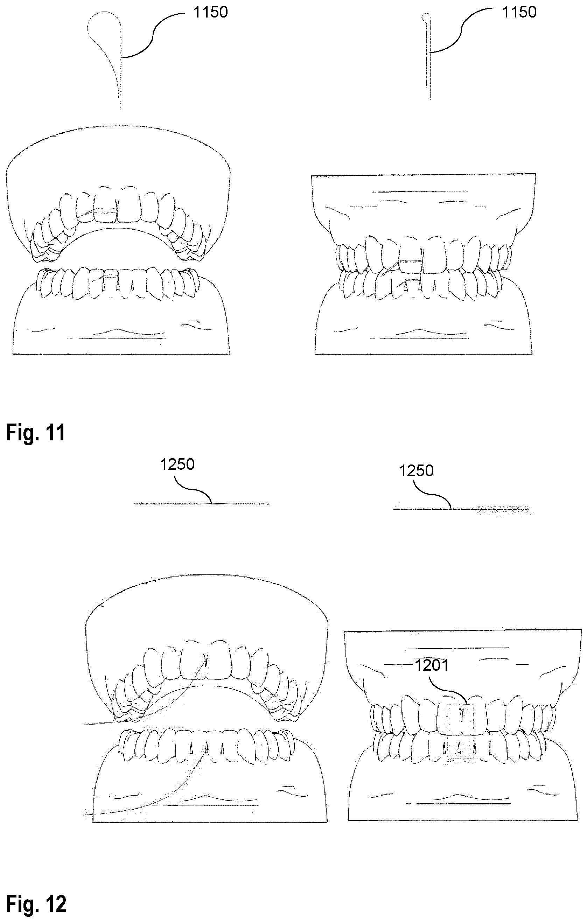

Another option for a fastening member 1150 is illustrated in Fig. 11, which can be used rather than floss. The fastening member 1150 is formed as a nitinol wire. Nitinol is a shape memory-memory alloy that changes its shape depending on the temperature. More specifically the nitinol wire has a first shape at a first temperature (temperature outside the mouth). In this configuration, the fastening member 1150 can be placed around one or more teeth. Here, a loop of nitinol wire is placed around a front tooth (left illustration in Fig. 11). When the fastening member 1150 reaches a certain temperature inside the mouth, the loop closes such that the fastening member 1150 is secured on the tooth. The final tightened configuration is shown in the right illustration of Fig. 11.

Another example of a fastening member 1250 is shown in Fig. 12, which is in the form of swelling floss. More specifically, after contact with saliva, the floss swells at least along a portion of its length such that it is secured in an interdental space. The final tightened configuration (with a plate-like member 1201 as described above) is shown in the right illustration of Fig. 12.

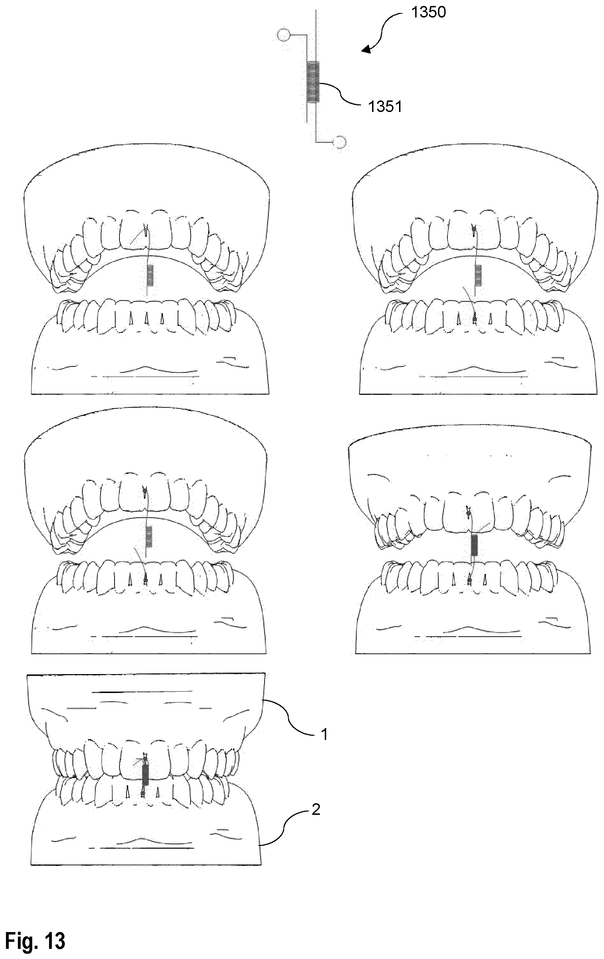

Still another example of a fastening member 1350 is shown in Fig. 13. More specifically, the steps of installing the fastening member 1350 are illustrated (from top left, followed by top right until the final phase at the bottom). One piece of the fastening member 1350 is provided with a tubular member 1351 comprising a braided structure, wherein a free end of another piece (or the other end of the same piece) is inserted into the tubular member 1351 that is configured to tighten when pulled. Such mechanism is known as "Chinese cuff" or "Chinese finger trap".

That means, while the tubular member 1351 is compressed during insertion of the free end and, thus, is open, the braided structure of the tubular member 1351 automatically contracts when the fastening member 1350 is tightened to thereby secure the relative position of the upper and lower jaws 1, 2.

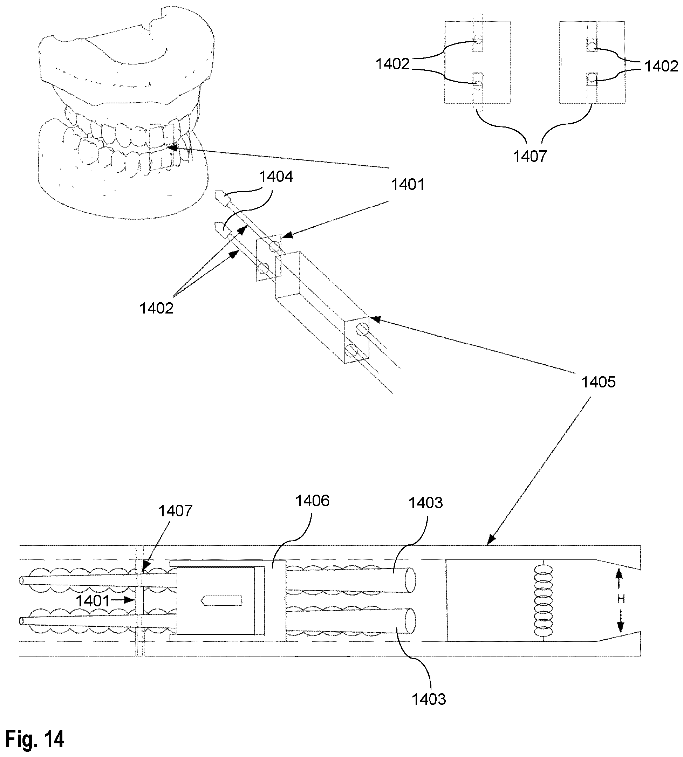

While the fastening members as described above, in particular the floss, may be preloaded in the device, it may also be possible to load the floss into the device only when installing the device in the mouth. in particular a locking plate 1401 fastened by means of floss lines 1402 to the front teeth in a similar way described above. In order to facilitate this procedure for the patient, a tool 1405 for may be provided as shown schematically in Fig. 14. Floss lines 1402 are inserted through elongated cone-shaped guiding elements 1403 extending through the tool 1405. The tool 1405 is held against the teeth and then pressed together (gripped on the right side in the lower drawing of Fig. 14) such that the guiding elements 1403 are pushed by a pushing element 1406 to protrude towards the teeth (left side in the lower drawing of Fig. 14). A line locker 1407 is provide that can displace the floss lines 1402 laterally so that they are locked in the locking plate 1401 when the tool is pressed together and can slide therethrough when the tool 1405 is released. The floss lines 1402 are pushed through respective interdental spaces and are held in place by means of anchoring members 1404 at their ends. The tool 1405 can then be released and removed from the teeth.

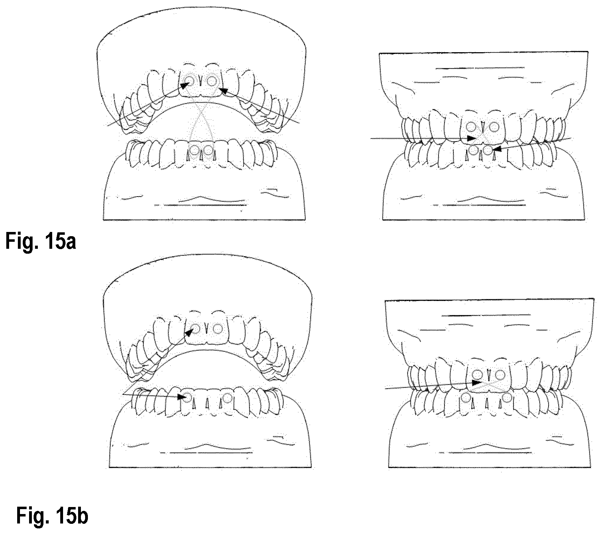

Another embodiment of a device 1500 for treating sleep apnea is shown in Figs. 15 and 15b. The device 1500 differs from the devices described above in that it is directly attached to the surface of the teeth 11, 12. More specifically, attachments 1501, 1502 are provided that attach to the surface of the teeth 11, 12, such as two front teeth 11 of the upper jaw 1 and two front teeth 12 of the lower jaw 2. Other teeth may be chosen for attaching the attachments, as shown in Fig. 15b compared to Fig. 15a. The attachments 1501, 1502 may be in the form of suction cups, which may allow easy attachment as well as easy removal. A fastening member 1550, such as an elastic band (e.g., rubber) brings the upper and lower jaws 1, 2 together and holds them in a relative position suitable for treating sleep apnea. Another option (not shown) can be to wrap an elastic band around the patient's chin rather than having attachments on teeth of the lower jaw.

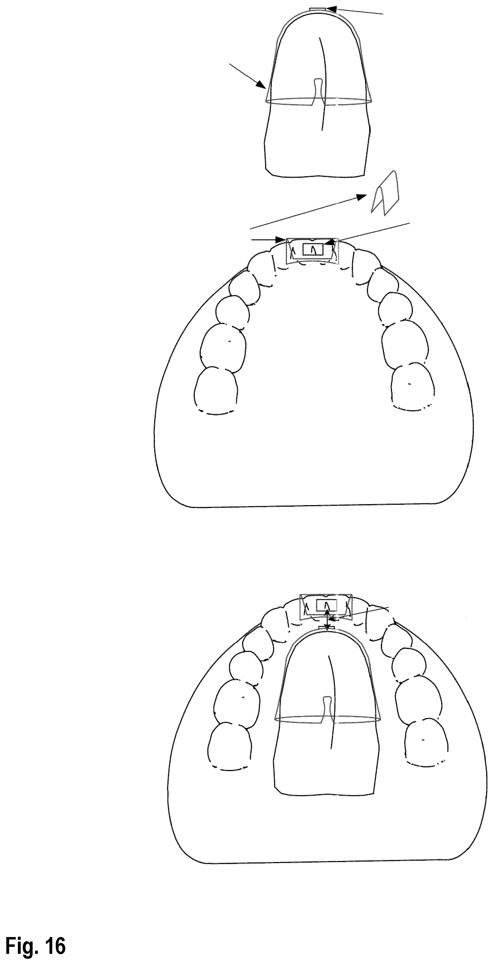

Fig. 16 shows another embodiment of a device 1600 for treating sleep apnea (components for the lower jaw 2 are not shown here). The device 1600 includes a tongue holding portion 1660 that is configured to hold the tongue 20 in a forward position to prevent the tongue 20 from obstructing the airway. The tongue holding portion 1660 comprises a "sleeve" (or "jacket") made of a flexible material (e.g., silicone) that is placed on the tip of the tongue 20 and held on the tongue 20 by a suction force. On its tip, the tongue holding portion 1660 has a magnet 1661 (e.g., embedded). The lower portion 1602 of the device 1600 (i.e., the portion configured to be placed on teeth 12 of the lower jaw 2) also has a magnet 1603 such that the tongue 20 can be maintained in a forward position by a magnet force.

While above at least one exemplary embodiment of the present invention has been described, it has to be noted that a great number of variations thereto exists. Furthermore, it is appreciated that the described exemplary embodiments only illustrate non-limiting examples of how the present invention can be implemented and that it is not intended to limit the scope, the application or the configuration of the herein-described apparatuses.

## Claims

1. A device for treating sleep apnea (100), the device comprising an upper portion (101), a lower portion (102), the upper portion configured to be placed on at least one tooth of the upper jaw of a patient, the lower portion being configured to be placed on at least one tooth of the lower jaw of the patient, the device further comprising at least one fastening member, the fastening member being configured to engage at least one interdental space of each of the upper and lower jaws to fasten the upper and lower portions to the teeth of the upper and lower jaws, respectively, **characterised in that** the device further comprises an intermediate portion (103), which is configured to couple the upper (101) and lower portions (102) together when placed on the teeth of the upper and lower jaws of the patient to maintain a relative position of the upper and lower jaws.

2. The device of claim 1, wherein the upper and lower portions each have at least one securing means for securing the fastening member to the upper and lower portions, respectively.

3. The device of claim 2, wherein the securing means comprises at least one of an opening, an eye, a pin, and a hook (212, 213) for engagement with the fastening member.

4. The device of any one of the preceding claims, wherein the fastening member comprises at least one piece of floss.

5. The device of any one of the preceding claims, wherein the fastening member comprises one or more strands of floss (420, 421), one or more loops of floss, or a combination thereof, wherein the fastening member comprises at least one piece of floss separately for each of the upper and lower portions, at least one piece of floss together for both of the upper and lower portions, or a combination thereof.

6. The device of any one of the preceding claims, wherein the upper and lower portions are formed as dental splints that are each configured to receive teeth of the upper jaw and lower jaw, respectively.

7. The device of claim 6, wherein the splints (301, 302) each have a retaining structure on an inner side thereof, the inner side facing the at least one tooth when placed on the tooth with the retaining structure engaging a surface of the at least one tooth.

8. The device of claim 7, wherein the retaining structure has at least one of one or more ridges, barbs, knobs, and pins.

9. The device of any one of the preceding claims, wherein the upper portion, the lower portion and the intermediate portion are formed as an integral piece.

10. The device of any one of the preceding claims, wherein the intermediate portion comprises at least one elongate connection member connecting the upper and lower portions.

11. The device of any one of claims 1 to 8, wherein the upper and lower portions are formed as separate pieces, and wherein the intermediate portion comprises a first portion attached to the upper portion and a second portion attached to the lower portion, the first and second portions being separate from each other and each accommodating a magnet (1603) such that the first and second portions are attachable to each other by magnetic force.

12. The device of any one of the preceding claims, further comprising a locking member (216) configured to engage the intermediate portion or the fastening member to secure the relative position of the upper and lower jaws.

13. The device of any one of the preceding claims, wherein the fastening member has an anchoring mechanism for anchoring in an interdental space.

14. The device of any one of the preceding claims, further comprising a tongue holding portion that is attached or attachable to at least one of the upper and lower portions, the tongue holding portion being configured to be placed on at least a portion of the patient's tongue to hold the tongue and prevent the tongue from moving towards the patient's throat.

15. The device of any one of the preceding claims, further comprising at least one sensor (220) for measuring at least one parameter in the patient's mouth.

## Patentansprüche

1. Vorrichtung zur Behandlung von Schlafapnoe (100), wobei die Vorrichtung einen oberen Abschnitt (101) und einen unteren Abschnitt (102) umfasst, wobei der obere Abschnitt so konfiguriert ist, dass er auf mindestens einem Zahn des Oberkiefers eines Patienten platziert werden kann, wobei der untere Abschnitt so konfiguriert ist, dass er auf mindestens einem Zahn des Unterkiefers des Patienten platziert werden kann, wobei die Vorrichtung ferner mindestens ein Befestigungselement umfasst, wobei das Befestigungselement so konfiguriert ist, dass es in mindestens einen Zahnzwischenraum sowohl des Ober- als auch des Unterkiefers eingreift, um den oberen bzw. den unteren Abschnitt an den Zähnen des Ober- bzw. des Unterkiefers zu befestigen, **dadurch gekennzeichnet, dass** die Vorrichtung ferner einen Zwischenabschnitt (103) umfasst, der so konfiguriert ist, dass er den oberen (101) und den unteren Abschnitt (102) miteinander koppelt, wenn er auf den Zähnen des Ober- und des Unterkiefers des Patienten platziert wird, um eine relative Position des Ober- und des Unterkiefers aufrechtzuerhalten.

2. Vorrichtung nach Anspruch 1, wobei der obere und der untere Abschnitt jeweils mindestens ein Sicherungsmittel zum Sichern des Befestigungselements an dem oberen bzw. dem unteren Abschnitt aufweisen.

3. Vorrichtung nach Anspruch 2, wobei das Sicherungsmittel mindestens eines von einer Öffnung, einer Öse, einem Stift und einem Haken (212, 213) zum Eingriff mit dem Befestigungselement umfasst.

4. Vorrichtung nach einem der vorhergehenden Ansprüche, wobei das Befestigungselement mindestens ein Stück Zahnseide (420, 421) umfasst.

5. Vorrichtung nach einem der vorhergehenden Ansprüche, wobei das Befestigungselement einen oder mehrere Stränge von Zahnseide, eine oder mehrere Schleifen von Zahnseide (420, 421) oder eine Kombination davon umfasst, wobei das Befestigungselement mindestens ein Stück Zahnseide separat für jeden des oberen und des unteren Abschnitts, mindestens ein Stück Zahnseide zusammen für sowohl den oberen als auch den unteren Abschnitt oder eine Kombination davon umfasst.

6. Vorrichtung nach einem der vorhergehenden Ansprüche, wobei der obere und der untere Abschnitt als Zahnschienen ausgebildet sind, die jeweils konfiguriert sind, Zähne des Oberkiefers bzw. des Unterkiefers aufzunehmen.

7. Vorrichtung nach Anspruch 6, wobei die Schienen (301, 302) jeweils eine Haltestruktur auf einer Innenseite davon aufweisen, wobei die Innenseite dem mindestens einen Zahn zugewandt ist, wenn sie auf dem Zahn platziert ist, wobei die Haltestruktur eine Oberfläche des mindestens einen Zahns in Eingriff nimmt.

8. Vorrichtung nach Anspruch 7, wobei die Haltestruktur mindestens eines von einem oder mehreren Rippen, Widerhaken, Noppen und Stiften aufweist.

9. Vorrichtung nach einem der vorhergehenden Ansprüche, wobei der obere Abschnitt, der untere Abschnitt und der Zwischenabschnitt als ein integrales Stück ausgebildet sind.

10. Vorrichtung nach einem der vorhergehenden Ansprüche, wobei der Zwischenabschnitt mindestens ein längliches Verbindungselement umfasst, das den oberen und den unteren Abschnitt verbindet.

11. Vorrichtung nach einem der Ansprüche 1 bis 8, wobei der obere und der untere Abschnitt als separate Stücke ausgebildet sind und wobei der Zwischenabschnitt einen ersten Abschnitt, der an dem oberen Abschnitt befestigt ist, und einen zweiten Abschnitt, der an dem unteren Abschnitt befestigt ist, umfasst, wobei der erste und der zweite Abschnitt voneinander getrennt sind und jeweils einen Magneten (1603) aufnehmen, so dass der erste und der zweite Abschnitt durch Magnetkraft aneinander befestigt werden können.

12. Vorrichtung nach einem der vorhergehenden Ansprüche, ferner umfassend ein Verriegelungselement (216), das so konfiguriert ist, dass es in den Zwischenabschnitt oder das Befestigungselement eingreift, um die relative Position des Ober- und des Unterkiefers zu sichern.

13. Vorrichtung nach einem der vorhergehenden Ansprüche, wobei das Befestigungselement einen Verankerungsmechanismus zum Verankern in einem Zahnzwischenraum aufweist.

14. Vorrichtung nach einem der vorhergehenden Ansprüche, ferner umfassend einen Zungenhalteabschnitt, der an mindestens einem des oberen und des unteren Abschnitts befestigt ist oder befestigt werden kann, wobei der Zungenhalteabschnitt so konfiguriert ist, dass er auf mindestens einem Abschnitt der Zunge des Patienten platziert werden kann, um die Zunge zu halten und zu verhindern, dass sich die Zunge in Richtung des Halses des Patienten bewegt.

15. Vorrichtung nach einem der vorhergehenden Ansprüche, ferner umfassend mindestens einen Sensor (220) zum Messen mindestens eines Parameters im Mund des Patienten.

## Revendications

1. Dispositif pour traiter l'apnée du sommeil (100), le dispositif comprenant une partie supérieure (101), une partie inférieure (102), la partie supérieure étant configurée pour être placée sur au moins une dent de la mâchoire supérieure d'un patient, la partie inférieure étant configurée pour être placée sur au moins une dent de la mâchoire inférieure du patient, le dispositif comprenant en outre au moins un élément de fixation, l'élément de fixation étant configuré pour venir en prise avec au moins un espace interdentaire de chacune des mâchoires supérieure et inférieure pour fixer les parties supérieure et inférieure aux dents des mâchoires supérieure et inférieure, respectivement, **caractérisé en ce que** le dispositif comprend en outre une partie intermédiaire (103), qui est configurée pour coupler les parties supérieure (101) et inférieure (102) ensemble lorsqu'elles sont placées sur les dents des mâchoires supérieure et inférieure du patient pour maintenir une position relative des mâchoires supérieure et inférieure.

2. Dispositif selon la revendication 1, dans lequel les parties supérieure et inférieure ont chacune au moins un moyen de fixation pour fixer l'élément de fixation aux parties supérieure et inférieure, respectivement.

3. Dispositif selon la revendication 2, dans lequel le moyen de fixation comprend au moins l'un parmi une ouverture, un oeillet, une broche et un crochet (212, 213) pour venir en prise avec l'élément de fixation.

4. Dispositif selon l'une quelconque des revendications précédentes, dans lequel l'élément de fixation comprend au moins une pièce de fil dentaire (420, 421).

5. Dispositif selon l'une quelconque des revendications précédentes, dans lequel l'élément de fixation comprend un ou plusieurs brins de fil, une ou plusieurs boucles de fil (420, 421), ou une combinaison de ceux-ci, dans lequel l'élément de fixation comprend au moins une pièce de fil séparément pour chacune des parties supérieure et inférieure, au moins une pièce de fil ensemble pour les deux parties supérieure et inférieure, ou une combinaison de celles-ci.

6. Dispositif selon l'une quelconque des revendications précédentes, dans lequel les parties supérieure et inférieure sont formées comme des attelles dentaires qui sont chacune configurées pour recevoir des dents de la mâchoire supérieure et de la mâchoire inférieure, respectivement.

7. Dispositif selon la revendication 6, dans lequel les attelles (301, 302) ont chacune une structure de retenue sur un côté interne de celles-ci, le côté interne faisant face à l'au moins une dent lorsqu'elle est placée sur la dent avec la structure de retenue mettant en prise une surface de l'au moins une dent.

8. Dispositif selon la revendication 7, dans lequel la structure de retenue a au moins l'un parmi une ou plusieurs crêtes, barbes, boutons et broches.

9. Dispositif selon l'une quelconque des revendications précédentes, dans lequel la partie supérieure, la partie inférieure et la partie intermédiaire sont formées comme une pièce d'un seul tenant.

10. Dispositif selon l'une quelconque des revendications précédentes, dans lequel la partie intermédiaire comprend au moins un élément de raccordement allongé raccordant les parties supérieure et inférieure.

11. Dispositif selon l'une quelconque des revendications 1 à 8, dans lequel les parties supérieure et inférieure sont formées comme des pièces séparées, et dans lequel la partie intermédiaire comprend une première partie fixée à la partie supérieure et une seconde partie fixée à la partie inférieure, les première et seconde parties étant séparées l'une de l'autre et chacune logeant un aimant (1603) de sorte que les première et seconde parties peuvent être fixées l'une à l'autre par une force magnétique.

12. Dispositif selon l'une quelconque des revendications précédentes, comprenant en outre un élément de verrouillage (216) configuré pour venir en prise avec la partie intermédiaire ou l'élément de fixation pour fixer la position relative des mâchoires supérieure et inférieure.

13. Dispositif selon l'une quelconque des revendications précédentes, dans lequel l'élément de fixation a un mécanisme d'ancrage pour l'ancrage dans un espace interdentaire.

14. Dispositif selon l'une quelconque des revendications précédentes, comprenant en outre une partie de maintien de langue qui est fixée ou peut être fixée à au moins l'une des parties supérieure et inférieure, la partie de maintien de langue étant configurée pour être placée sur au moins une partie de la langue du patient pour maintenir la langue et empêcher la langue de se déplacer vers la gorge du patient.

15. Dispositif selon l'une quelconque des revendications précédentes, comprenant en outre au moins un capteur (220) pour mesurer au moins un paramètre dans la bouche du patient.
